Europäisches Patentamt

European Patent Office

Office européen des brevets

(11) Veröffentlichungsnummer: **0 049 514**
A1

## (12) EUROPÄISCHE PATENTANMELDUNG

(21) Anmeldenummer: 81107976.3

(22) Anmeldetag: 06.10.81

(51) Int. Cl.³: **A 61 M 15/00**

(30) Priorität: 08.10.80 DE 3037917

(43) Veröffentlichungstag der Anmeldung:
14.04.82 Patentblatt 82/15

(84) Benannte Vertragsstaaten:
AT BE CH DE FR GB IT LI LU NL SE

(71) Anmelder: **Byk Gulden Lomberg Chemische Fabrik GmbH**
**Byk-Gulden-Strasse 2**
**D-7750 Konstanz(DE)**

(72) Erfinder: **Schweitzer, Manfred**
**Erlenweg 7**
**D-8956 Germaringen(DE)**

(54) Inhaliergerät.

(57) Die Anwendungseigenschaften eines bekannten Inhaliergerätes mit atemgesteuerter Wirkstofffreisetzung werden durch schall- und bewegungsdämpfende Maßnahmen am Auslösemechanismus verbessert.

Fig 1

EP 0 049 514 A1

Die Erfindung betrifft ein Inhaliergerät mit atemgesteuerter Wirkstofffreisetzung mit verbesserten Anwendungseigenschaften.

In der DE-OS 20 55 734 ist ein Inhaliergerät beschrieben, das beim Einwirken von Atemsog über ein Dosierventil eine abgemessene Menge eines
Medikaments aus einem Aerosolbehälter an einen Patienten verabfolgt.
Das Aerosol wird automatisch zu Beginn der Einatmung frei, so daß der
Wirkstoff zum richtigen Zeitpunkt für die Inhalation zur Verfügung
steht. Ein entsprechendes Gerät, das den Wirkstoff Hexoprenalin enthält, ist inzwischen unter der Bezeichnung Dosier-Aerosol-Synchron-
Inhalator im Handel.

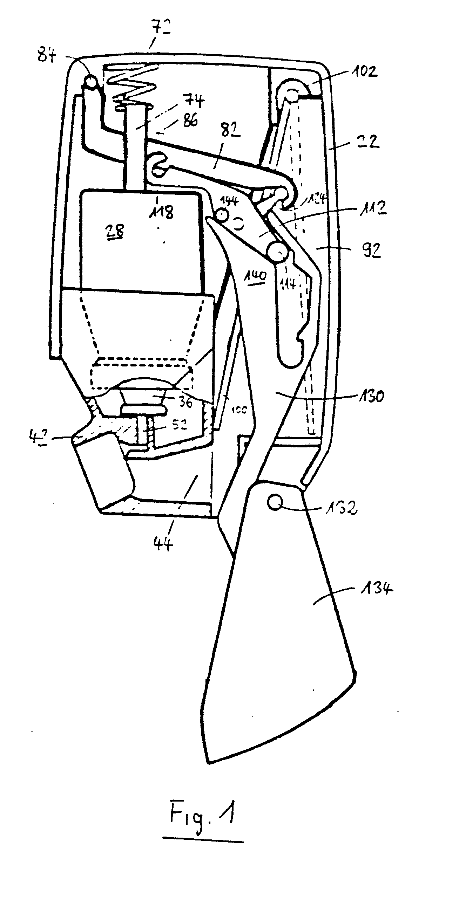

Die Funktionsweise des Inhaliergeräts wird nachfolgend anhand von
Fig. 1 beschrieben. Eine genaue Beschreibung ist    der DE-OS 20 55 734
zu entnehmen. Fig. 1 zeigt einen Längsschnitt durch den Inhalator und
gibt nur die funktionellen Teile wieder. Die für die einzelnen Teile
verwendeten Bezeichnungsweisen und Bezugszeichen entsprechen soweit wie
möglich der DE-OS 20 55 734.

In einem Gehäuse 22 mit einem bei 132 schwenkbaren aufklappbaren Deckel
134 befindet sich ein Aerosolbehälter 28 mit Dosierventil 36, das in
einem Mundstück 42 steckt. Vom Deckel 134 erstreckt sich ein gabelförmiger Auslöser 130 nach oben in die Auslöserkammer 92. Gabelteil 140
des Auslösers 130 greift an einem Stift 144 einer um 114 schwenkbaren
Klinke 112 an. Beim Schließen von Deckel 134 bringt Gabelteil 140 die
Klinke 112 von der in Fig. 1 gezeigten Endstellung in eine zweite Endstellung, in der diese mit Haken 118 sperrend an der einseitig abgeflachten Welle 102 angreift. Zugleich wirkt der Hebel 124 auf den um 84
schwenkbaren Füllhebel 82 und schwenkt ihn gegen den Druck der Feder
72 nach oben, wobei der Stössel 74 nach oben verschoben wird. Die um
die Welle 102 schwenkbare Steuerklappe 100 wird gleichzeitig in ihre
rechte Endstellung geschwenkt (gestrichelt gezeichnet). Beim Anlegen
von Atemsog am Mundstück 42 wird die Steuerklappe 100 durch den über
Kanal 44 auf sie wirkenden Unterdruck in ihre linke Endstellung gebracht, wobei der Eingriff von Haken 118 der Klinke 112 an Welle 102
gelöst wird, wodurch Klinke 112, Füllhebel 82 und Stössel 74 durch den
Druck der Feder 72 in die in Fig. 1 gezeigte Stellung gebracht werden.

BAD ORIGINAL

Dabei wird Aerosolbehälter 28 gegen den Sitz des Ventilschafts 52 gedrückt, wobei die im Dosierventil 36 abgemessene Menge Aerosolzubereitung freigesetzt wird. Durch Schließen von Deckel 134 ist der Inhalator wieder betriebsbereit.

Beim Gebrauch dieses Inhalators nach der DE-OS 20 55 734 stellte sich heraus, daß von vielen Patienten das Klickgeräusch und der am Mundstück verspürbare Bewegungsimpuls beim Betätigen des Auslösemechanismus als sehr irritierend empfunden wird. Viele Patienten reagieren in Erwartung der mit dem Auslösen verbundenen, für sie unangenehmen Empfindungen verkrampft, was sich negativ auf die Verteilung des Wirkstoffs im Atemtrakt auswirkt, weil nicht tief und locker genug eingeatmet wird. Außerdem verunmöglicht das laute Klickgeräusch eine diskrete Anwendung des Inhaliergeräts.

Aufgabe der vorliegenden Erfindung war es, diesen erheblichen Nachteil des Inhalators mit möglichst geringem Aufwand zu beseitigen.

Erfindungsgemäß gelöst wurde diese Aufgabe dadurch, daß die Klinke 112 auf ihrer dem Aerosolbehälter 28 zugewandten Schmalseite 112 a mit einer Auflage 112 b aus einem dämpfenden Material ausgestattet wird und/oder der Boden 28 a des Aerosolbehälters 28 mit einer Auflage 28 b aus dämpfendem Material ausgestattet wird und/oder Stössel 74 an seinem an den Aerosolbehälter 28 anliegenden Ende mit einem Träger 74 a ausgestattet ist, der zumindest im Bereich unterhalb der Klinke 112 mit dämpfendem Material 74 b belegt ist.

Es zeigte sich, daß durch diese wenig aufwendigen dämpfenden Maßnahmen die Akzeptanz des Inhaliergerätes stark erhöht wurde.

Gegenstand der Erfindung ist daher ein Inhaliergerät gemäß der DE-OS 20 55 734, dadurch gekennzeichnet, daß schall- und bewegungsdämpfende Maßnahmen vorgesehen sind.

Fig. 1 zeigt schematisch das Inhaliergerät nach der DE-OS 20 55 734.

Fig. 2 bzw. Fig. 3 a, 3 b, 3 c zeigen Ausgestaltungen der erfindungsgemäß vorgeschlagenen schall- und bewegungsdämpfenden Maßnahmen.

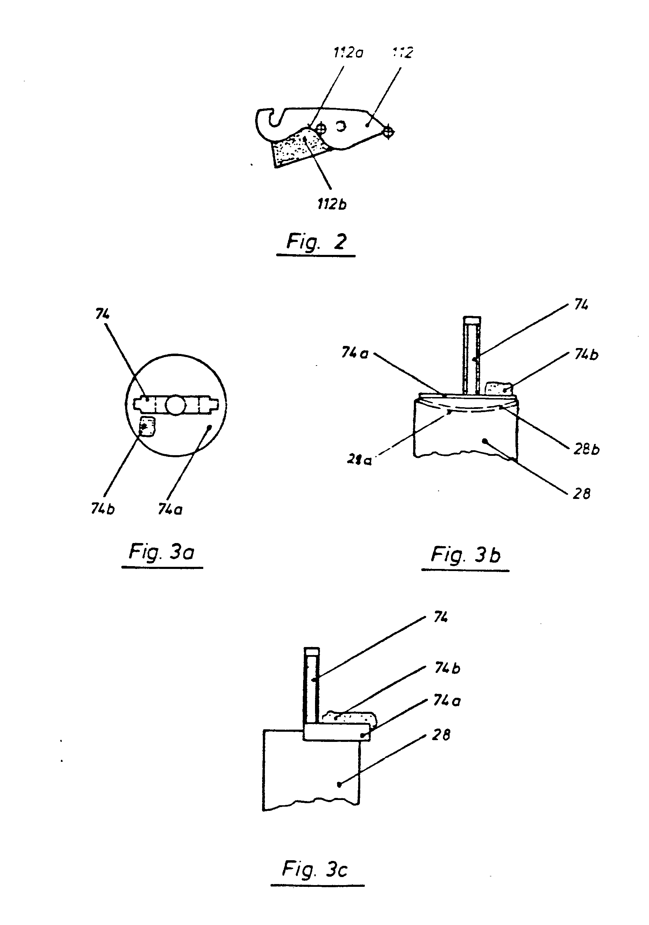

Eine bevorzugte Ausgestaltung besteht darin, daß die Klinke 112 auf ihrer Schmalseite 112 a mit einer Auflage aus dämpfendem Material ausgestattet ist. Als dämpfendes Material kommen viele weichelastische Materialien in Frage. Neben filz-, kork- und gummiartigen Materialien sind dies insbesondere Schaumkunststoffe. Fig. 2 zeigt Klinke 112 mit einer dämpfenden Auflage 112 b an der konkaven Schmalseite 112 a. Diese Auflage wird durch Kleben angebracht. Die Auswahl der jeweils in Frage kommenden Kleber ist dem Fachmann bekannt. Bei Schaumkunststoffen wird vorzugsweise eine Verbindung durch an sich bekanntes Kleben oder Schweißen, insbesondere Ultraschall- oder Hochfrequenzschweißen zwischen dämpfender Auflage 112 b und Klinke 112 hergestellt. Die Dicke der dämpfenden Auflage beträgt etwa 0,5 bis 10 mm, vorzugsweise 5 bis 8 mm.

Eine weitere Ausgestaltung besteht darin, daß der Boden 28 a des Aerosolbehälters 28 mit einer dämpfenden Auflage 28 b versehen wird. Als dämpfende Stoffe kommen dieselben wie bei der vorgenannten Ausgestaltung in Frage. Vorzugsweise wird der Boden 28 a nur in einem engen Bereich, der in etwa der Berührungsfläche mit der Schmalseite 112 a der Klinke 112 entspricht mit dämpfendem Material belegt. Hierbei ist es zweckmäßig, auf Mundstück 42 und Aerosolbehälter 28 Markierungen, insbesondere Farbmarkierungen, anzubringen, damit beim Einsetzen eines Aerosolbehälters 28 in den Inhalator die dämpfende Auflage 28 b auf dem Boden 28 a des Aerosolbehälters 28 zwangsläufig unterhalb der Klinke 112 zu liegen kommt. Alternativ ist es möglich Aerosolbehälter 28 und Mundstück 42 mit Führungseinrichtungen zu versehen, die nur ein bestimmtes Einsetzen eines Aerosolbehälters 28 erlauben.

Eine weitere Ausgestaltung besteht darin, daß der Stössel 74 an seinem dem Boden 28 a des Aerosolbehälters anliegenden Ende mit einem Träger 74 a ausgestattet ist, der zumindest im Bereich, der unterhalb der Klinke 112 zu liegen kommt mit einer Auflage 74 b aus dämpfendem Material belegt ist. Es kommen verschiedene Ausführungsformen des Trägers 74 a in Frage. Träger 74 a erstreckt sich beispielsweise, wie in Fig. 3 a in Aufsicht und in Fig. 3 b in Seitenansicht dargestellt, kreisförmig über den Boden 28 a des Aerosolbehälters 28 und ist vorzugsweise der Wölbung des Bodens 28 a angepaßt. Es ist aber auch möglich, den Träger 74 a nur halbkreisförmig oder streifenförmig auf der

BAD ORIGINAL

der Klinke 112 zugewandten Seite auszubilden. Es ist wunschgemäß auch möglich, den Träger 74 a, wie in Fig. 3 c schematisch dargestellt, kragenförmig über den Rand des Bodens 28 a des Aerosolbehälters 28 herabzuziehen, um somit die Führung des Aerosolbehälters 28 zu verbessern. Bei dieser Ausgestaltung kann die Auflage 74 b über die Kante des Trägers 74 a herabreichen. Träger 74 a ist vorzugsweise einstückig mit Stössel 74 ausgebildet. Je nach Stärke des Trägers 74 a kann es zweckmäßig sein, diesen auf seiner oberen, dem Boden 28 a abgewandten Seite mit Verstärkungsrippen zu versehen und/oder mit dem Stössel 74 durch Versteifungswände zu verbinden. Es ist auch möglich, Stössel 74 und Träger 74 a nicht einstückig auszuführen, sondern beispielsweise den Stössel 74 mit Zapfen zu versehen, sodaß er auf den Träger 74 a aufgesteckt und wunschgemäß beispielsweise durch Verschweissen unlösbar mit ihm verbunden werden kann. Um ein Abweichen von Auflage 74 b zur Seite zu verhindern, können seitlich der Auflage 74 b Begrenzungsrippen vorgesehen werden. Es ist zweckmäßig, die Höhe des Stössels 74 mit Träger 74 a identisch mit der des Stössels nach der DE-OS 20 55 734 bzw. des handelsüblichen Geräts zu wählen, womit eine Anpassung der übrigen Teile des Inhaliergeräts entbehrlich wird. Die Materialien, deren Stärke und Verbindung untereinander werden entsprechend der erstgenannten Ausgestaltung gewählt. Es ist auch möglich, die Auflage 74 b aus dämpfendem Material direkt am Stössel 74 im Schwenkbereich der Klinke 112 anzubringen, womit Träger 74 a weggelassen werden kann.

Es liegt im Rahmen dieser Erfindung, jeweils zwei oder alle drei Ausgestaltungen gemeinsam zu verwirklichen.

BAD ORIGINAL

Patentansprüche

1. Inhaliergerät mit atemgesteuerter Wirkstofffreisetzung nach DE-OS 20 55 734, dadurch gekennzeichnet, daß schall- und bewegungsdämpfende Maßnahmen vorgesehen sind.

2. Inhaliergerät nach Patentanspruch 1, dadurch gekennzeichnet, daß die Klinke (112) auf ihrer dem Aerosolbehälter (28) zugewandten Schmalseite (112 a) mit einer Auflage (112 b) aus dämpfendem Material ausgestattet ist und/oder der Boden (28 a) des Aerosolbehälters (28) mit einer Auflage (28 b) aus dämpfendem Material ausgestattet ist und/oder der Stössel (74) an seinem dem Aerosolbehälter (28) anliegenden Ende mit einem Träger (74 a) ausgestattet ist, der zumindest im Bereich unterhalb der Klinke (112) mit einer Auflage (74 b) aus dämpfendem Material belegt ist.

3. Inhaliergerät nach Patentanspruch 2, dadurch gekennzeichnet, daß als Auflage (112 b, 28 b bzw. 74 b) aus dämpfendem Material Schaumkunststoff verwendet wird.

4. Inhaliergerät nach Patentanspruch 3, dadurch gekennzeichnet, daß die Auflage (112 b, 28 b bzw. 74 b) aus dämpfendem Material durch Kleben oder Schweißen, insbesondere Ultraschall- oder Hochfrequenzschweißen, angebracht ist.

5. Inhaliergerät nach Patentanspruch 2, dadurch gekennzeichnet, daß der Träger (74 a) kreisförmig über dem Boden (28 a) des Aerosolbehälters (28) ausgebildet ist.

6. Inhaliergerät nach Patentanspruch 2, dadurch gekennzeichnet, daß der Träger (74 a) halbkreisförmig über dem Boden (28 a) auf der der Klinke (112) zugewandten Seite ausgebildet ist.

7. Inhaliergerät nach einem der Patentansprüche 5 oder 6, dadurch gekennzeichnet, daß der Träger (74 a) der Wölbung des Bodens (28 a) des Aerosolbehälters (28) angepaßt ist.

BAD ORIGINAL

8. Inhaliergerät nach einem der Patentansprüche 5, 6 oder 7, dadurch gekennzeichnet, daß der Träger (74 a) kragenförmig über den Rand des Bodens (28 a) des Aerosolbehälters (28) herabgezogen ist.

9. Inhaliergerät nach Patentanspruch 8, dadurch gekennzeichnet, daß die Auflage (74 b) über die Kante des Trägers (74 a) herabgezogen ist.

Fig. 1

'90 EP

10.81

*Fig. 2*

*Fig. 3a*

*Fig. 3b*

*Fig. 3c*

0049514

Europäisches Patentamt

**EUROPÄISCHER RECHERCHENBERICHT**

## EINSCHLÄGIGE DOKUMENTE

| Kategorie | Kennzeichnung des Dokuments mit Angabe, soweit erforderlich, der maßgeblichen Teile | betrifft Anspruch |
|---|---|---|
| AD | <u>DE - A - 2 055 734</u> (RIKER LABS. INC.) | 1 |
| | -- | |
| | <u>FR - A - 1 352 329</u> (EMW-BETRIEB VIBROSTOP, et al.)<br>* Ganzes Dokument * | 1-3 |
| | -- | |
| | <u>FR - A - 1 393 204</u> (P. RECHNER et al.)<br>* Ganzes Dokument * | 1-4 |
| | -- | |
| | <u>US - A - 2 869 913</u> (E. SCHLAGE)<br>* Die Figuren; Spalte 2, Zeilen 29-59 * | 1-3 |
| | -- | |
| | <u>DE - B - 1 130 324</u> (E. DOERPINGHAUS)<br>* Ganzes Dokument * | 1 |
| | -- | |
| | <u>GB - A - 355 618</u> (K. KAYE et al.)<br>* Ganzes Dokument * | 1 |
| | ---- | |

KLASSIFIKATION DER ANMELDUNG (Int. Cl.)

A 61 M   15/00

RECHERCHIERTE SACHGEBIETE (Int. Cl.)

A 61 M
E 05 B

KATEGORIE DER GENANNTEN DOKUMENTE

X von besonderer Bedeutung allein betrachtet
Y von besonderer Bedeutung in Verbindung mit einer anderen Veröffentlichung derselben Kategorie
A technologischer Hintergrund
O nichtschriftliche Offenbarung
P Zwischenliteratur
T der Erfindung zugrunde liegende Theorien oder Grundsätze
E älteres Patentdokument, das jedoch erst am oder nach dem Anmeldedatum veröffentlicht worden ist
D in der Anmeldung angeführtes Dokument
L aus andern Gründen angeführtes Dokument

& Mitglied der gleichen Patentfamilie, übereinstimmendes Dokument

Der vorliegende Recherchenbericht wurde für alle Patentansprüche erstellt

| Recherchenort<br>Den Haag | Abschlußdatum der Recherche<br>22-12-1981 | Prüfer<br>VEREECKE |
|---|---|---|

EPA form 1503.1   06.78

BAD ORIGINAL